(19) Europäisches Patentamt
European Patent Office
Office européen des brevets

(11) Veröffentlichungsnummer: **0 002 534**
**B1**

(12) # EUROPÄISCHE PATENTSCHRIFT

(45) Veröffentlichungstag der Patentschrift: **13.01.82**

(51) Int. Cl.³: **C 07 F 9/38, A 61 K 37/02**

(21) Anmeldenummer: **78101702.5**

(22) Anmeldetag: **15.12.78**

(54) **Aminosäurederivate, Verfahren zu deren Herstellung, Zwischenprodukte bei deren Herstellung und diese enthaltende pharmazeutische Präparate.**

(30) Priorität: **20.12.77 GB 5293877**
**01.11.78 GB 4272778**

(43) Veröffentlichungstag der Anmeldung:
**27.06.79 Patentblatt 79/13**

(45) Bekanntmachung des Hinweises auf die Patenterteilung:
**13.01.82 Patentblatt 82/2**

(84) Benannte Vertragsstaaten:
**BE CH DE FR IT LU NL SE**

(56) Entgegenhaltungen:
**DE - A - 2 602 193**
**DE - A - 2 632 188**
**DE - A - 2 730 524**
**DE - A - 2 732 454**

**Chemical Abstracts vol. 82, no. 21, 26 May 1975**
**Columbus, Ohio USA**
**J. HUBER et al "Synthesis and antimicrobial evaluation of N-D-alanyl-1-aminoethylphosphonic acid"**
**Seite 84, Spalte 1, Abstract Nr. 133460q**
**& J. Med. Chem. 1975, 18 (1) 106—8**
**I. Klages, Lehrbuch der organischen Chemie, Bd. III (1958), S.416—419**

(73) Patentinhaber: **F. HOFFMANN-LA ROCHE & CO. Aktiengesellschaft**
**CH-4002 Basel (CH)**

(72) Erfinder: **Atherton, Frank Ratcliffe**
**148 Parkway**
**Welwyn Garden City Herts. (GB)**
Erfinder: **Hall, Michael John**
**15 Connonsfield Road**
**Welwyn Herts. (GB)**
Erfinder: **Hassall, Cedric Herbert**
**"Trelech" Tewin Close Tewin Wood**
**Welwyn Herts. (GB)**
Erfinder: **Lambert, Robert Wilson**
**5 Grange Hill**
**Welwyn Herts. (GB)**
Erfinder: **Ringrose, Peter Stuart**
**41 The Limes**
**Harston Cambridgeshire (GB)**

(74) Vertreter: **Lederer, Franz, Dr. et al,**
**Patentanwälte Lederer, Meyer Lucile-Grahn-Strasse 22**
**D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 002 534 B1

# Aminosäurederivate, Verfahren zu deren Herstellung, Zwischenprodukte bei deren Herstellung und diese enthaltende pharmazeutische Präparate

Die vorliegende Erfindung betrifft neue Aminosäure derivate der allgemeinen Formel I

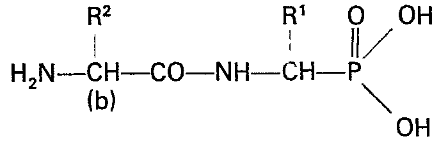

(I)

worin $R^1$ Wasserstoff oder Methyl und $R^2$ n-$C_{2-4}$-Alkyl oder 4-Guanidino-butyl darstellen, mit R-Konfiguration am mit (a) gekennzeichneten C-Atom (falls $R^1$ = Methyl) und L-Konfiguration am mit (b) gekennzeichneten C-Atom,

sowie pharmazeutisch verträgliche Salze davon.

Verbindungen der Formel I sind:

(1R)-1-[L-($\alpha$-Aminobutyryl)amino]-äthyl-phosphonsäure;
(1R)-1-(L-Norleucylamino)-äthylphosphonsäure;
(1R)-1-(L-Norvalylamino)-äthylphosphonsäure;
[L-($\alpha$-Aminobutyryl)amino]-methylphosphonsäure;
1-(L-Norleucylamino)-methylphosphonsäure;
1-(L-Norvalylamino)-methylphosphonsäure;
(1R)-1-(L-Homoarginylamino)-äthylphosphonsäure;
1-(L-Homoarginylamino)-methylphosphonsäure.

Die Aminosäure derivate der vorliegenden Erfindung, d.h. die Verbindungen der Formel I und deren pharmazeutisch verträgliche Salze, können erfindungsgemäss durch Kondensation einer Verbindung der allgemeinen Formel II

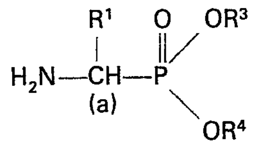

(II)

worin $R^1$ wie in Formel I definiert ist und $R^3$ und $R^4$ Wasserstoff oder niedere Alkylschutzgruppen darstellen; mit R-Konfiguration am C-Atom (a), wenn $R^1$ Methyl ist,

mit einer geschützten $\alpha$-Aminosäure der allgemeinen Formel III

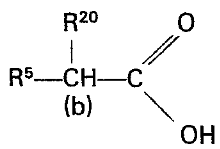

(III)

worin $R^{20}$ dasselbe wie $R^2$ in Formel 1 oder geschütztes 4-Guanidino-butyl und $R^5$ eine geschützte Aminogruppe darstellt; mit L-Konfiguration am C-Atom (b),

gefolgt von Abspaltung vorhandener Schutzgruppen aus dem Kondensationsprodukt und, gewünschtenfalls, Ueberführung in ein pharmazeutisch verträgliches Salz, hergestellt werden.

Als geschützte Aminogruppen, wie sie durch $R^5$ definiert sein sollen, kommen die aus der Peptidchemie wohlbekannten Gruppen in Frage, vorzugsweise die durch Aralkoxycarbonyl-reste, besonders den Benzyloxycarbonyl-rest, oder den tert. Butoxycarbonyl-rest, geschützten Aminogruppen. Die Aminogruppe kann jedoch auch durch Formyl-, Trityl-, Trifluoracetyl- oder 2-(Biphenyl)-isopropyloxycarbonyl-Reste geschützt sein oder sie kann in Form einer Phthalimidogruppe vorliegen.

Die erfindungsgemässe Kondensation einer Verbindung der Formel II mit einer geschützten $\alpha$-Aminosäure der Formel III kann nach den an sich bekannten Methoden der Peptidchemie ausgeführt werden, beispielsweise nach der gemischten Anhydrid-, Azid-, aktivierte Ester-, Säurechlorid-, Carbodiimid- oder der EEDQ-Methode. (EEDQ=1-Aethoxycarbonyl-2-äthoxy-1,2-dihydro-chinolin).

So kann eine Verbindung II mit einer geschützten $\alpha$-Aminosäure III kondensiert werden, in der die Carboxygruppe als gemischte Anhydridgruppe (aus der Anhydrid-bildung mit einer organischen oder anorganischen Säure) vorliegt. Eine solchermassen geschützte Säure III kann mit einer tertiären Base, wie einem Tri-niederalkyl)-amin, z.B. Triäthylamin, oder N-Aethylmorpholin, in einem inerten Lösungsmittel (z.B. Tetrahydrofuran, 1,2-Dimethoxy-äthan, Dichlormethan, Toluol, Petroläther) behandelt und das dabei erhaltene Salz mit einem geeigneten Chlorformiat, z.B. einem nieder-Alkyl-chloroformiat, wie Aethylchlorformiat oder Isobutylchlorformiat) bei niedriger Temperatur umgesetzt werden. Das so erhaltene gemischte Anhydrid wird dann vorzugsweise in situ mit einer Verbindung II umgesetzt.

Nach einer anderen Methode kann die Verbindung II mit einer geschützten α-Aminosäure III kondensiert werden, in der die Carboxygruppe als Säureazidgruppe vorliegt. Diese Kondensation wird vorzugsweise bei niedriger Temperatur in einem inerten organischen Lösungsmittel, wie Dimethylformamid oder Aethylacetat, ausgeführt.

Nach einer weiteren Methode kann die Verbindung II mit einer geschützten α-Aminosäure III kondensiert werden, in der die Carboxygruppe in Form einer aktivierten Estergruppe vorliegt, z.B. als 4-Nitrophenyl-, 2,4,5-Trichlorphenyl- oder N-Hydroxysuccimidester-gruppe. Diese Kondensation wird vorzugsweise in einem inerten Lösungsmittel durchgeführt, wie wässrigem Dimethylformamid oder, wenn $R^3$ und $R^4$ in einer Verbindung II beide für nieder-Alkyl stehen, auch in einem niederen Alkanol, wie wässrigem Aethanol.

Die verbindung II kann auch mit einer geschützten α-Aminosäure III kondensiert werden, in der die Carboxygruppe in Form einer Säurechloridgruppe vorliegt. In diesem Fall erfolgt die Kondensation vorzugsweise in Gegenwart einer Base und bei niedriger Temperatur.

Die Kondensation einer Verbindung II mit einer geschützten α-Aminosäure III kann auch in Gegenwart eines Carbodiimids (z.B. Dicyclohexylcarbodiimid) oder von EEDQ erfolgen. Diese Kondensation kann in einem inerten organischen Lösungsmittel (z.B. Methylenchlorid oder einem niederen Alkanol, wie Methanol, Aethanol) bei Raumtemperatur oder darunter durchgeführt werden.

Die Abspaltung der Schutzgruppe(n) aus dem Kondensationsprodukt kann nach an sich bekannten Methoden durchgeführt werden, d.h. nach Methoden, wie sie tatsächlich ausgeübt werden oder wie sie in der Literatur beschrieben sind. So kann beispielsweise eine Aralkoxycarbonylgruppe (wie Benzyloxycarbonyl), die tert. Butoxycarbonylgruppe oder die 2-Biphenyl-isopropyloxycarbonylgruppe durch Hydrolyse abgespalten werden, z.B. durch Behandlung mit HBr in Eisessig. Eine Aralkoxycarbonylgruppe (wie Benzyloxycarbonyl) kann auch mittels Hydrogenolyse abgespalten werden, z.B. in Gegenwart von Palladium/Tierkohle oder von Platinoxid. Die tert.-Butoxycarbonyl- oder die 2-Biphenyl-isopropyloxycarbonylgruppe kann auch unter Verwendung von HCl in Dioxan abgespalten werden. Eine Tritylgruppe kann z.B. durch Behandlung mit verdünnter Essigsäure abgespalten werden.

Eine Phthalimidogruppe kann mittels Hydrazinolyse in eine Aminogruppe übergeführt werden. Niederalkyl-Schutzgruppen, wie sie u.a. durch $R^3$ und $R^4$ definiert werden, können durch Behandlung mit HBr in Eisessig abgespalten werden, oder mit Trimethylchlorsilan oder Trimethylbromsilan gefolgt von wässriger Hydrolyse. Es versteht sich, dass bei Anwesenheit von mehr als einer Schutzgruppe, die Abspaltung der Schutzgruppen je nach der Art dieser Gruppen in einem einzigen Schritt oder in mehreren Schritten ausgeführt werden kann. Vorzugsweise wird man jedoch Schutzgruppen verwenden, die sich in einem einzigen Schritt abspalten lassen.

Die Verbindungen der Formel I sind amphoter und bilden demzufolge pharmazeutisch verträgliche Salze, sowohl mit pharmazeutisch verträglichen starken Säuren (z.B. Salzsäure, Bromwasserstoffsäure, Paratoluolsulfonsäure) wie mit Basen (z.B. Natrium- oder Kaliumhydroxid).

Die Ausgangsmaterialien der Formeln II und III sind bekannte Verbindungen oder können in Analogie zur Herstellung bekannter Verbindung hergestellt werden.

Die durch Kondensation einer Verbindung II mit einer Verbindung III zunächst erhaltenen Kondensationsprodukte der allgemeinen Formel IV

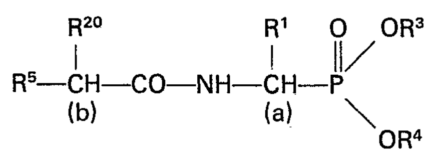

(IV)

worin $R^1$, $R^3$ und $R^4$ wie in Formel II definiert sind; $R^5$ und $R^{20}$ wie in Formel III definiert sind und die Konfigurationen an den C-Atomen (a) und (b) die im Zusammenhang mit Formel I angegebenen sind,

sind neu und bilden ebenfalls Gegenstand der Erfindung.

Die erfindungsgemässen Aminosäurederivate der Formel I sind antibakteriell gegen einen breiten Bereich von gram-positiven und gram-negativen Bakterien aktiv, wie Escherichia coli, Staphylococcus aureus, Serratia marcescens, Klebsiella aerogenes, Streptococcus faecalis, Haemophilus influenzae, Bacillus subtilis, Salmonella typhimurium, Proteus mirabilis, Pseudomonas aeroginosa und Shigella sonnei. Zusätzlich potenzieren die erfindungsgemässen Peptidderivate die Aktivität von Antibiotica, unter Einschluss von Penicillin- und Cephalosporin-Antibiotica und D-Cycloserin. Als Antibiotica, die durch die erfindungsgemässen Peptidderivate potenziert werden, können die folgenden erwähnt werden: Amoxycillin, Cephadrine, Cephalothin, Cephalexin, Carbenicillin, Ampicillin, Penicillin G, Sulbenicillin, Cephazolin, Cefoxitin, Rifampicin, [(R) - 1 - (2 - furoyloxy) - 3 - methylbutyl]penicillin, (6R) - 6 - [<(Hexahydro - 1H - azepin - 1 - yl) - methylen>amino]penicillansäure,(Pivaloyloxy)methyl - (6R) - 6 - [<(hexahydro - 1H - azepin - 1 - yl)methylen>amino]penicillinat, Cephamandole, Cephaloridin, Cephaloglycin, Phenethicillin, Methicillin, Propicillin, Ticaracillin, Amoxycillin-Argininsalz, Phosphonomycin, Vancomycin und Kanamycin.

Die erfindungsgemässen Verbindungen der Formel I stehen strukturell und wirkungsmässig Verbindungen der deutschen Offenlegungsschriften Nos. 26 02 193 und 27 32 454 nahe. Gegenüber der wirkungsmässig insgesamt besten Verbindung dieser Anmeldungen, (1R)-1-(L-Alanylamino)-aethylphosphonsäure, weisen die neuen Verbindungen überraschenderweise gegen eine Anzahl pathogener Mikroorganismen Vorteile im Sinne geringerer minimaler Hemmkonzentrationen auf.

Die vorliegende Erfindung umfasst auch pharmazeutische Präparate, die zusammen mit einem pharmazeutisch verträglichen Trägermaterial ein erfindungsgemässes Aminosäurederivat und erwünschtenfalls zusätzlich ein Antibioticum enthalten.

Als Trägermaterial kommt jedes feste oder flüssige Trägermaterial in Frage, das mit den erfindungsgemässen Aminosäurederivaten und mit den gegebenenfalls anwesenden Antibiotica verträglich ist und das sich für therapeutische Verabreichung eignet. Das Trägermaterial kann ein organisches oder anorganisches Material sein, das sich für enterale (z.B. orale) oder parenterale Verabreichung eignet. Beispiele solcher Trägermaterialien sind Wasser, Gelatine, Laktose, Stärke, Magnesiumstearat, Talk, vegetabile Oele, Gummi arabicum, Polyalkylenglykole etc. Die pharmazeutischen Präparate können in feste Form (z.B. Tabletten, Dragées, Suppositorien oder Kapseln) oder in flüssige Form gebracht werden (z.B. Lösungen, Suspensionen oder Emulsionen). Sie können den üblichen pharmazeutischen Operationen, wie Sterilisation, unterworfen werden und können Zusatzstoffe, wie Konservierungs-, Stabilisierungs-, Netz- oder Emulgiermittel, Salze zur Veränderung des osmotischen Druckes oder Puffer, enthalten. Bei Verwendung eines Puffers kann das pH des pharmazeutischen Präparates natürlich innerhalb des aus der pharmazeutischen Praxis wohlbekannten Bereiches variieren.

Wenn das erfindungsgemässe pharmazeutische Präparat ein Aminosäurederivat und ein Antibioticum enthält, kann das Gewichtsverhältnis des Peptidderivats zum Antibioticum innerhalb weiter Grenzen variieren. Im allgemeinen wird das genannte Gewichtsverhältnis zwischen 1:100 und 100:1 liegen. Bevorzugt sind Gewichtsverhältnisse zwischen 1:64 bis 64:1 und besonders zwischen 1:16 bis 16:1.

Die tägliche Dosis des allein oder in Kombination mit einem Antibioticum verabreichten Aminosäurederivats wird innerhalb weiter Grenzen variieren, in Abhängigkeit von Faktoren, wie dem gewählten Aminosäurederivat, gegebenenfalls dem gewählten Antibioticum, der Verabreichungsart und der zu behandelnden Infektion. Wenn z.B. ein Aminosäurederivat allein verabreicht wird, kann die tägliche Dosis für orale Verabreichung bis zu ungefähr 2000—4000 mg, und die tägliche Dosis für parenterale Verabreichung bis zu ungefähr 800—2000 mg, betragen. Wenn ein Aminosäurederivat in Kombination mit einem Antibioticum verabreicht wird, kann die tägliche Dosis für orale Verabreichung bis zu ungefähr 750—1500 mg der Kombination von Aminosäurederivat und Antibioticum und eine tägliche Dosis für parenterale Verabreichung bis zu ungefähr 200—2000 mg der Kombination betragen. Es versteht sich, dass die tägliche Dosis in einer einzigen Dosis oder in Teilen verabreicht werden kann und dass die erwähnten Dosierungen sowohl nach oben wie nach unten variiert werden können, je nach individuellen Erfordernissen und in Anpassung an die Besonderheiten der tatsächlichen Situation, wie sie vom behandelnden Arzt festgestellt werden.

## Beispiel 1

1.1 Herstellung des Ausgangsmaterials:

5,0g (48 mMol) L-$\alpha$-Aminobuttersäure wurden in 24 ml 2N Natronlauge gelöst. Die Lösung wurde auf 0°C gekühlt. 12,3g (72mMol) Benzylchlorformiat und 18 ml (72 mMol) 4N Natronlauge wurden alternierend und portionenweise unter Rühren im Verlaufe einer halben Stunde zugegeben, und zwar so, dass die Temperatur 10°C nicht überstieg und das pH bei ca. 11 gehalten wurde. Die Mischung wurde dann auf Raumtemperatur gebracht, über Nacht gerührt und mit 50 ml Diäthyläther extrahiert. Die organischen und wässerigen Phasen wurden getrennt. Die wässrige Phase wurde mit 5N Salzsäure auf pH 2 eingestellt, was ein öliges Gemisch ergab. Dieses Gemisch wurde mit zwei 75 ml Portionen Diäthyläther extrahiert. Die Extrakte wurden über Natriumsulfat getrocknet und eingedampft, was 9,6g eines farblosen Oels ergab. Dieses Oel wurde in 10 ml Aethylacetat gelöst. 100 ml Petroläther (Sdp. 40—60°C) wurden zugegeben. Die erhaltene ölige Lösung wurde bei 0°C stehen gelassen. So wurde ein weisser kristalliner Niederschlag erhalten, der abfiltriert, mit Petroläther gewaschen und getrocknet wurde. Man erhielt 8.40g (73%) N-Benzyloxycarbonyl-L-$\alpha$-aminobuttersäure vom Smp. 76—78°C; $[\alpha]_D^{20} = -10,9°$; $[\alpha]_{365}^{20} = -20,3°$ (c= 1% in Aethanol).

1.2 Das Verfahren:

1.2.1 8,30g (35 mMol) N-Benzyloxycarbonyl-L-$\alpha$-aminobuttersäure und 4,02g (35 mMol) N-Hydroxysuccinimid wurden unter Rühren in 75 ml Dimethoxyäthan gelöst. Die Lösung wurde auf 0°C gekühlt. 7,94g (38,5 mMol) Dicyclohexylcarbodiimid wurden zugegeben. Das Gemisch wurde bei 0°C 2 Stunden gerührt und dann bei 0°C über Nacht stehen gelassen. Ein festes Nebenprodukt wurde abfiltriert. Das filtrat wurde eingedampft. Man erhielt so den N-Benzyloxycarbonyl-L-$\alpha$-aminobuttersäure-N-hydroxysuccinimidester in Form eines steifen Gummis, welcher ohne Reinigung weiter verwendet wurde.

1.2.2 4,38g (35 mMol) (1R)-1-Amino-äthylphosphonsäure wurden in einer Mischung von 40 ml Wasser, 7,07g (70 mMol) Triäthylamin und 40 ml Dimethylformamid unter Rühren gelöst. Die Lösung wurde auf 0°C gekühlt und rasch eine Lösung von 35 mMol N-Benzyloxycarbonyl-L-$\alpha$-aminobuttersäure-N-hydroxysuccinimidester in 40 ml Dimethylformamid zugetropft. Das Gemisch wurde bei 0°C gerührt und dann über Nacht unter Rühren auf Raumtemperatur gebracht. Zur Entfernung einer kleinen Menge Festsubstanz wurde das Gemisch dann filtriert. Dass filtrat wurde unter Oelpumpenvacuum eingedampft. Der Rückstand wurde in einem Gemisch von 30 ml Methanol und 10 ml Wasser gelöst und durch eine Kolonne eines Kationen-Austauscherharzes (B.D.H, Zerolit 225, SRC 13, $RSO_3H$; 150g frisch regeneriert im Säurecyclus) geleitet, wobei die Elution mit demselben Lösungsmittel ausgeführt wurde. Das saure Eluat wurde gesammelt und zu einer leicht gummiartigen, weissen Festsubstanz eingedampft. Diese Festsubstanz wurde mit 100 ml Diäthyläther verrieben, worauf der Diäthyläther abdekantiert wurd. Die resultierende Festsubstanz wurde in einem Gemisch von 100 ml Methanol und 50 ml Wasser gelöst und die Lösung mit 4N wässrigem Benzylamin (Titer 8,0 ml, Theorie 8,75 ml) auf pH 4,5 titriert. Das Gemisch verfestigte sich gegen das Ende der Titration. Der Niederschlag wurde abfiltriert, mit Wasser gewaschen und getrocknet. Man erhielt so 8,84g des Monobenzylaminsalzes von (1R)-1-[(N-Benzyloxycarbonyl-L-$\alpha$-aminobutyryl)amino]-äthylphosphonsäure von Smp. 228—231°C (Zersetzung); $[\alpha]_D^{20} = -31,3°C$; $[\alpha]_{365}^{20} = -105°$ (c= 1% in Eisessig).

1.2.3 8,7g (19,3 mmol) des Monobenzylaminsalzes von (1R)-1-[N-Benzyloxycarbonyl-L-$\alpha$-aminobutyryl)amino]-äthylphosphonsäure wurden 6 Stunden bei Raumtemperatur mit einem Gemisch von 20 ml 45% HBr in Eisessig und 8 ml Eisessig verrührt. Nach Zugabe von 100 ml Aether resultierte ein gummiartiger Niederschlag, welcher durch Dekantieren mit 100 ml Diäthyläther gewaschen und dann in 100 ml Methanol gelöst wurde. Die Lösung wurde gerührt. Nach Zugabe von zwei 5 ml Portionen Propylenoxid (pH ca. 5) erhielt man einen weissen Niederschlag. Das Gemisch wurde über Nacht stehen gelassen, der Niederschlag abfiltriert, mit Methanol und Diäthyläther gewaschen und getrocknet. Man erhielt 4,05g rohe (1R)-1-(L-$\alpha$-Aminobutyrylamino)-äthylphosphonsäure vom Smp. 298°C (Zers.). Durch Umkristallisation aus einer Mischung von 750 ml Wasser und 1500 ml Aethanol wurde ein weisser kristalliner Niederschlag erhalten, der mit Aethanol, dann mit Diäthyläther gewaschen und schliesslich im Vacuum getrocknet wurde. Man erhielt so 3,58g (88%) (1R)-1-(L-$\alpha$-Aminobutyrylamino)-äthylphosphonsäure vom Smp. 296—298°C (Zers.); $[\alpha]_D^{20} = -33,1°$; $[\alpha]_{365}^{20} = -145°$ (c= 1N NaOH, frisch hergestellt).

Beispiel 2

2.1 Herstellung des Ausgangsmaterials:

5,0g (42,5 mMol) L-Norvalin wurden mit 10,2g (60 mMol) Benzylchlorformiat und Natriumhydroxid behandelt, wie im Beispiel 1.1 beschrieben. Das rohe ölige Produkt wurde aus einer Mischung von 10 ml Diäthyläther und 20 ml Petroläther (Sdp. 40—60°C) kristallisiert. Man erhielt 8,2g (77%) N-Benzyloxycarbonyl-L-norvalin von Smp. 85—87°C; $[\alpha]_D^{20} = -9,9°$; $[\alpha]_{365}^{20} = -26,5°$ (c= 1% in Aethanol).

2.2 Das Verfahren:

2.2.1. Auf die im Beispiel 1.2.1 beschriebene Art erhielt man aus 8,1g (32 mMol) N-Benzyloxycarbonyl-L-norvalin, 3,68g (32 mMol) N-Hydroxysuccinimid und 7,21g (35 mMol) Dicyclohexylcarbodiimid, nach Verreiben des öligen Produktes mit 25 ml Aethanol, ein weisses kristallines Produkt. Zugabe von 25 ml Petroläther (Sdp. 40—60°C) und Filtration ergaben 9,82g N-Benzyloxycarbonyl-L-norvalin-N-hydroxysuccinimidester, welcher direkt im nächsten Schritt verwendet wurde. Eine gereinigte Probe schmolz bei 95—97°C und zeigte eine optische Drehung $[\alpha]_D^{20} = -35,1°$ (c = 1% in Aethanol).

2.2.2 Auf die im Beispiel 1.2.2 beschriebene Art erhielt man aus 9,82g (28 mMol) N-Benzyloxycarbonyl-L-norvalin-N-hydroxysuccinimidester und 3,75g (30 mMol) (IR)-1-Aminoäthylphosphonsäure (jedoch unter Verwendung eines Gemisches von 150 ml Methanol und 30 ml Wasser zum Verreiben, anstelle von Diäthyläther) und 7,61g des Monobenzylaminsalzes von (1R)-1-[(N-Benzyloxycarbonyl-L-norvalyl)amino]-äthylphosphonsäure vom Smp. 225—230°C (Zers.); $[\alpha]_D^{20} = -29,9°C$; $[\alpha]_{365}^{20} = -98,8°$ (c= 1% in Essigsäure).

Eindampfen des Filtrats und Behandlung des Rückstandes mit 100 ml warmem Wasser, gefolgt von Filtration, Waschen mit Aethanol und dann mit Diäthyläther ergab eine zweite Portion von 1,99g des Monobenzylaminsalzes von (1R)-1-[(N-Benzyloxycarbonyl-L-norvalyl)amino]-äthylphosphonsäure vom Smp. 231—234°C (Zers.); $[\alpha]_D^{20} = -29,7°$; $[\alpha]_{365}^{20} = -99,2°$ (c = 1% in Essigsäure). Totalausbeute = 9,60g (73%).

2.2.3 Auf die im Beispiel 1.2.3 beschriebene Art erhielt man aus 9.0g (19 mMol) des Monobenzylaminsalzes von (1R)-1-[(N-Benzyloxycarbonyl-L-norvalyl)amino]-äthylphosphonsäure nach Kristallisation aus einer Mischung von 80 ml Wasser und 160 ml Aethanol, 3,54g (82%) (1R)-1-(L-Norvalylamino)-äthylphosphonsäure vom Smp. 260—262°C (Zers.); $[\alpha]_D^{20} = -19,5°$ $[\alpha]_{365}^{20} = -75,3°$ (c= 1% in Wasser).

## Beispiel 3

3.1 Herstellung des Ausgangsmaterials:

Auf die im Beispiel 1.1 beschriebene Art wurden 5.0g (38 mMol) L-Norleucin mit 9,7g (57 mMol) Benzylchlorformiat und Natriumhydroxid behandelt. Das erhaltene N-Benzyloxycarbonyl-L-norleucin wurde in Form eines Oels isoliert, welches ohne Kristallisation weiter verwendet wurde.

3.2 Das Verfahren:

3.2.1 Auf die im Beispiel 1.2.1 beschriebene Art erhielt man aus ca. 38 mMol N-Benzyloxycarbonyl-L-norleucin, 4,38g (38 mMol) N-Hydroxysuccinimid und 8,65g (42 mMol) Dicyclohexylcarbodiimid, nach Behandlung des rohen, öligen Produkts mit 100 ml Diäthyläther, 7,47g N-Benzyloxycarbonyl-L-norleucin-N-hydroxysuccinimidester als kristallinen Festkörper mit Smp. 82—84°C; $[\alpha]_D^{20} = -20,7°$ (c= 1% in Aceton).

3.2.2 Auf die im Beispiel 1.2.2 beschriebene Art (jedoch unter Ausführung des Ionenaustauscherschrittes in Methanol/Wasser 5:1, anstelle von Methanol/Wasser 3:1) erhielt man aus 7,40g (20, 5 mMol) N-Benzyloxycarbonyl-L-norleucin-N-hydroxysuccinimidester und 2,56g (20,5 mMol) (1R)-1-Aminoäthyl-phosphonsäure, nach Eindampfen und Verreiben des rohen Produkts mit Aether, eine Festsubstanz, die nach Behandlung mit warmem Wasser, Kühlung und Filtration 5,64g (74%) (1R)-1-[(N-Benzyloxycarbonyl-L-norleucyl)amino]-äthylphosphonsäure vom Smp. 192—194°C (Zers.) ergab; $[\alpha]_D^{20} = -37,8°$; $[\alpha]_{365}^{20} = -126,5°$ (c= 0,5% in Essigsäure).

3.2.3 Auf die im Beispiel 1.2.3 beschriebene Art erhielt man aus 5,20g (14,0 mMol) (1R)-1-[(N-Benzyloxycarbonyl-L-norleucyl)amino]-äthylphosphonsäure nach Kristallisation aus einem Gemisch von 70 ml Wasser und 280 ml Aethanol, 3,02g (91%) (1R)-1-(L-Norleucylamino)-äthylphosphonsäure vom Smp. 254—256°C (Zers.); $[\alpha]_D^{20} = -18,6°$; $[\alpha]_{365}^{20} = -70,1°$ (c= 1% in Wasser).

## Beispiel 4

4.1. Auf die im Beispiel 1.2.2 und Beispiel 2.2.2 beschriebene Art (aber unter Ausführung der Titration mit Benzylamin in einem Gemisch von 250 ml Methanol und 50 ml Wasser) erhielt man aus 17,4g (50 mMol) N-Benzyloxycarbonyl-L-norvalin-N-hydroxysuccinimidester und 5,55g (50 mMol) Aminomethylphosphonsäure, 17,3g rohes Monobenzylaminsalz des gewünschten Produkts von Smp. 195—198°C (Zers.) (1. Portion). Eindampfen des Filtrats und Kristallisation des Rückstandes aus einer Mischung von 80 ml Methanol und 20 ml Wasser ergaben weitere 2,19g von im wesentlichen reinem Produkt (Dünnschichtchromatographie) mit Smp. 180—185°C (Zers.). Totalausbeute: 19,5g (86%).

Umkristallisation von 0,5g der 1. Portion aus 10 ml warmen Wasser ergab 0,39g reines Monobenzylaminsalz von (N-Benzyloxycarbonyl-L-norvalyl)amino-methylphosphonsäure vom Smp. 203—205°C (Zers.); $[\alpha]_D^{20} = -8,2°$; $[\alpha]_{365}^{20} = -21,3°$ (c= 0,5% in Essigsäure).

4.2 Auf die im Beispiel 1.2.3 beschriebene Art erhielt man aus 18,9g (42 mMol) Monobenzylaminsalz von (N-Benzyloxycarbonyl-L-norvalyl)amino-methylphosphonsäure nach Kristallisation aus einem Gemisch von 80 ml Wasser und 160 ml Aethanol, 6,65g (75%) L-(Norvalylamino)-methylphosphonsäure vom Smp. 273—275°C; $[\alpha]_D^{20} = +61,2°$; $[\alpha]_{365}^{20} = +224°$ (c= 0,5% in Wasser).

## Beispiel 5

N-Benzyloxycarbonyl-L-norvalin (45,4g 0,181 M) wurde in Methylendichloridlösung (200 ml) gerührt, während Dimethylaminomethylphosphonat-hydrochlorid (31,76g, 0,181 M) zugeführt wurde. Die resultierende Suspension wurde auf −12°C gekühlt, während trockenes Triäthylamin (23,3 ml, 0,181 M) zugefügt wurde. Nach vollendeter Zugabe wurde die kalte Mischung 15 Minuten gerührt, worauf E.E.D.Q (56, 8g, 0,23 M) in Methylendichlorid (100 ml) rasch zugegeben wurde. Diese Mischung wurde Kalt 2 Stunden und dann bei Raumtemperatur über das Wochenende gerührt.

Die Mischung wurde mit Wasser (100 ml), dann mit 1N HCl (4 mal mit je 100 ml) gewaschen. Die gesamten sauren Waschflüssigkeiten wurden mit Methylendichlorid (2 mal 50 ml) zurückextrahiert. Die vereinigte organische Lösung wurde wieder mit Wasser (100 ml) und schliesslich mit 15%-iger $KHCO_3$-Lösung (3 mal 100 ml) gewaschen, dann über wasserfreiem Natriumsulfat getrocknet. Die Lösung wurde filtriert und eingedampft. Das verbleibende Oel wurde wieder eingedampft (aus Benzol), wobei ein Rückstand von 78,13g resultierte.

Das Oel wurde mit wasserfreiem Aether (200 ml) verrieben, wobei es sich im Aether löste. Sehr bald trat Kristallisation ein. Die Lösung wurde 1 Stunde an die Kälte gestellt, die Festsubstanz abfiltriert, mit Aether gewaschen und bis zur Gewichtskonstanz im Vacuum getrocknet. Man erhielt 58,2g eines Produkts vom Smp. 77—80°C. Dieses wurde in warmem Aethylacetat (200 ml) aufgenommen und die Lösung filtriert. Zum gekühlten Filtrat wurde Aether (200 ml) zugefügt. Die Mischung wurde angeimpft und über Nacht kalt gestellt. Die Festsubstanz wurde abfiltriert, mit Aether gewaschen und im Vacuum getrocknet. Es wurden 50,64g eines Produkts vom Smp. 82—84°C erhalten.

37,2g [(N-Benzyloxycarbonyl-L-norvalyl)-amino]-dimethylphosphonat wurden in 45%-igem HBr in Essigsäure (120 ml) 5 Stunden gerührt. Anfänglich war die $CO_2$-Entwicklung sehr rapid. Aether (500 ml) wurde zugefügt, worauf sich ein Oel ausschied. Das Gemisch wurde 40 Minuten gerührt und dann ruhen gelassen. Der Aether wurde abdekantiert und das Oel 2 mal mit Aether gewaschen (jeweils mit 500 ml). Das Oel wurde in Methanol (300 ml) aufgenommen, dann die Lösung unter Zugabe von Propylenoxid (40 ml) gerührt. Nach 5 Minuten begann das Produkt auszukristallisieren. Die Mischung wurde auf pH 4 eingestellt und über Nacht kalt gestellt. Der Feststoff wurde abfiltriert, dann gut mit Methanol und Aether gewaschen und im Vacuum getrocknet. Man erhielt 20,13g eines Produktes vom Smp. 288—9°C (Zers.). Dieses wurde in warmem Wasser (200 ml) aufgenommen und filtriert. Zum Filtrat wurden 400 ml Aethanol gegeben. Das nach Stehenlassen auskristallisierte Produkt wurde 2 Stunden kalt gestellt. Der Feststoff wurde abfiltriert, mit Aethanol und dann mit Aether gewaschen und über Phosphorpentoxid im Vacuum getrocknet. Man erhielt so 16,66g (L-Norvalylamino)-methylphosphonsäure von Smp. 293—4°C (Zers.); $[\alpha]_D^{20} = +62,7°$ (c= 0,5% in Wasser).

## Beispiel 6

6.1 L-Nitro-homoarginin (2,33g, 10 mMol, Smp. 227—230°C, Zers.) wurde mit 3,41g (20 mMol) Benzylchlorformiat und Natriumhydroxid behandelt, wie im Beispiel 1.1 beschrieben. Das rote ölige Produkt wurde aus der angesäuerten Lösung mit Aethylacetat (2 mal mit je 100 ml) extrahiert (anstatt mit Diäthyläther). Das rote ölige Produkt, N-Benzyloxycarbonyl-$\omega$-nitro-L-homoarginin, wurde nach Trocknen über Natriumsulfat und Eindampfen als gummiartige Substanz erhalten und so ohne weitere Reinigung im folgenden Schritt verwendet.

6.2 Auf die im Beispiel 1.2.1 beschriebene Art, jedoch unter Verwendung von Dimethylformamid (20 ml) als Lösungsmittel, erhielt man aus ca. 4,2g (ungefähr 10 mMol) rohem N-Benzyloxycarbonyl-$\omega$-nitro-L-homoarginin, 1,15g (10 mMol) N-Hydroxysuccinimid und 2,27g (11 mMol) Dicyclohexylcarbodiimid, nach Entfernung von 1,73g Dicyclohexyl harnstoff und Einengen des Filtrats, rohen N-Benzyloxycarbonyl-$\omega$-nitro-L-homoarginin-N-hydroxysuccinimidester als gelbes Oel, welches im nächsten Schritt ohne weitere Reinigung verwendet wurde.

6.3 Auf die im Beispiel 1.2.2 beschriebene Art wurden ca 10 mMol N-Benzyloxycarbonyl-$\omega$-nitro-L-homoarginin-N-hydroxysuccinimidester und 1,25g (10 mMol) (1R)-1-Aminoäthylphosphonsäure umgesetzt, wobei jedoch die Behandlung mit dem Ionenaustauscherharz $RSO_3H$ in 5:1 $MeOH:H_2O$ erfolgte, statt mit 3:1. Das saure Eluat wurde eingedampft und zwischen Wasser (300 ml) und Aethylacetat (150 ml) verteilt, wobei etwas unlösliches Material erhalten wurde. Dieses wurde abfiltriert und getrocknet (1,36g vom Smp. 190—193°C (Zers.). (1. Portion).

Die Lösungsmittelschichten des Filtrats wurden getrennt, die wässrige Schicht wurde zur Trockene eingedampft und der Rückstand mit Aceton (50 ml) verrieben, wobei ein weisser Niederschlag resultierte. Dieser Niederschlag wurde abfiltriert, mit Aceton gewaschen und getrocknet. Man erhielt so weitere 1,43g (1R)-1-[(N-Benzyloxycarbonyl-$\omega$-nitro-L-homoarginyl)-amino]-äthylphosphonsäure vom Smp. 184—8°C (Zers.). (2. Portion). Die Portionen 1 und 2 wurden ohne weitere Reinigung im nächsten Schritt verwendet.

6.4 Auf die im Beispiel 1.2.3 beschriebene Art erhielt man aus 2,7g (ca. 5,7 mMol) (1R)-1-[(N-Benzyloxycarbonyl-$\omega$-nitro-L-homoarginyl)-amino]-äthylphosphonsäure, nach Kristallisation aus einem Gemisch von 15 ml Wasser und 150 ml Aethanol, 1,23g (1R)-1-(l-$\omega$-Nitro-homoarginylamino)-äthylphosphonsäure vom Smp. ca. 190° (Zers.); $[\alpha]_D^{20} = 11,2°$; $[\alpha]_{365}^{20} = 43,2°$ (c= 0,51% in Wasser). Totalausbeute: 137g (71%).

6.5 1,2g (3,5 mMol) (1R)-1-(L-$\omega$-Nitro-homoarginylamino)-äthylphosphonsäure wurden in 50 ml Wasser aufgenommen. Dann wurden 0,35g 10%-ige Pd/Tierkohle zugesetzt. Das Gemisch wurde bei Raumtemperatur und unter Druck bis zur Beendigung der $H_2$—Aufnahme hydriert (ungefähr 5 Stunden). Das Gemisch wurde zur Entfernung des Katalysators filtriert und das Filtrat eingedampft. Der Gummiartige Rückstand wurde in 15 ml kaltem Wasser aufgenommen, die Lösung filtriert und mit 120 ml Aethanol behandelt. Der erhaltene Niederschlag wurde nach 1 Stunde abfiltriert. Man erhielt so 0,76g (1R)-1-(L-Homoarginylamino)-äthylphosphonsäure vom Smp. ca. 195°C (Zers.); $[\alpha]_D^{20} = -11,9°$ (c= 0,5% in Wasser).

## Beispiel 7

7.1 Auf die im Beispiel 1.2.2 beschriebene Art erhielt man aus einer Lösung von 45 mMol N-Benzyloxycarbonyl-L-$\alpha$-aminobuttersäure-N-hydroxysuccinimidester in 40 ml Dimethylformamid und 4,44g (40 mMol) Aminomethylphosphonsäure (unter Verwendung von Methanol:Wasser im Verhältnis von 4:1 statt 2:1), 14,1g des Monobenzylaminsalzes von [(N-Benzyloxycarbonyl-L-$\alpha$-aminobutyryl)amino]-methylphosphonsäure vom Smp. 185—195°C (Zers.). Durch Eindampfen des Filtrats und Umkristallisation des Rückstandes aus einem Gemisch von 50 ml Wasser und 50 ml Methanol erhielt man weitere 1,6g Produkt als weissen kristallinen Niederschlag von Smp. 205—208°C

(Zers.); $[\alpha]_D^{20} = -6{,}1°$; $[\alpha]_{365}^{20} = -21{,}1°$ (c= 0,51% in Essigsäure).

7.2 Auf die im Beispiel 1.2.3 beschriebene Art erhielt man aus 15,6g (ca 36 mMol) des Monobenzylaminsalzes von [(N-Benzyloxycarbonyl-L-$\alpha$-aminobutyryl)amino]-methylphosphonsäure 5,92g rohes Produkt vom Smp. 253—255°C (Zers.). Umkristallisation aus einem Gemisch von 60 ml Wasser und 120 ml Aethanol ergaben 4,93g (L-$\alpha$-Aminobutyrylamino)-methylphosphonsäure vom Smp. 263—265°C (Zers.); $[\alpha]_D^{20} = +57{,}0°$; $[\alpha]_{365}^{20} = +212°$ (c= 0,51% in Wasser).

Beispiel 8

8.1 Auf die im Beispiel 3.2.2 beschreibene Art erhielt man aus 2.52g (7 mMol) N-Benzyloxycarbonyl-L-norleucin-N-hydroxysuccinimidester und 0.78g (7 mMol) Aminomethylphosphonsäure, nach Rühren mit 100 ml Aceton, 0,56g des Monobenzylaminsalzes von [(N-Benzyloxycarbonyl-L-norleucyl)amino]-methylphosphonsäure vom Smp. 185—189°C (Zers.). Dabei wurden die Ionenaustauschreaktion in Methanol/Wasser 2:1, anstatt 5:1, und die Titration des sauren Eluats direkt mit Benzylamin, also unter Vermeidung von Eindampfen, Behandlung mit Aether und nachfolgendem Wiederauflösen in Methanol/Wasser ausgeführt.

Durch Eindampfen des Filtrats und Verreiben mit 50 ml Diäthyläther erhielt man weitere 1,9g Produkt vom Smp. 170—180°C (Zers.).

8.2 Auf die im Beispiel 1.2.3 beschriebene Art erhielt man aus 2.40g (ca. 5,2 mMol) des Monobenzylaminsalzes von [(N-Benzyloxycarbonyl-L-norleucyl)amino]-methylphosphonsäure 1,04g rohes Produkt vom Smp. 263—266°C (Zers.). Durch Umkristallisation aus einem Gemisch von 20 ml Wasser und 120 ml Aethanol erhielt man 0,88g (L-Norleucylamino)-methylphosphonsäure vom Smp. 272—274°C (Zers.); $[\alpha]_D^{20} = +63{,}4°$; $[\alpha]_{365}^{20} = +231°$ (c= 0,5% in Wasser).

Das folgende Beispiel illustriert eine typische galenische Darreichungsform:

Beispiel A

Eine parenterale Formulierung kann aus folgenden Bestandteilen zusammengesetzt sein:

| | *per 1000 ml* |
|---|---|
| Peptidderivat der Formel I | 50,0g |
| Chlorkresol | 1,0g |
| Eisessig | 1,2g |
| NaOH-Lösung (0,1 N) q.s. | ad pH 4,5 |
| Wasser für Injektionszwecke | ad 1000 ml |

**Patentansprüche**

1. Aminosäurederivate der allgemeinen Formel I

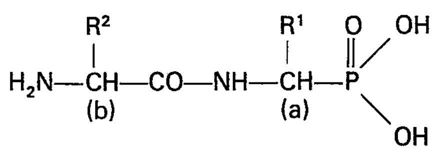

(I)

worin $R^1$ Wasserstoff oder Methyl und $R^2$ n-$C_{2-4}$-Alkyl oder 4-Guanidinobutyl bedeuten, mit R-Konfiguration am mit (a) gekennzeichneten C-Atom (wenn $R^1$ = Methyl) und L-Konfiguration am mit (b) gekennzeichneten C-Atom,

sowie deren pharmazeutisch verträgliche Salze.

2. (1R)-1-[L-($\alpha$-Aminobutyryl)-amino]-äthylphosphonsäure und deren pharmazeutisch verträgliche Salze.

3. (1R)-1-(L-Norleugylamino)-äthylphosphonsäure und deren pharmazeutisch verträgliche Salze.

4. (1R)-1-(L-Norvalylamino)-äthylphosphonsäure und deren pharmazeutisch verträgliche Salze.

5. (L-$\alpha$-Aminobutyrylamino)-methylphosphonsäure und deren pharmazeutisch verträgliche Salze.

6. (L-Norleucylamino)-methylphosphonsäure und deren pharmazeutisch verträgliche Salze.

7. (L-Norvalylamino)-methylphosphonsäure und deren pharmazeutisch verträgliche Salze.
8. (1R)-(L-Homoarginylamino)-äthylphosphonsäure und deren pharmazeutisch verträgliche Salze.
9. (L-Homoarginylamino)-methylphosphonsäure und deren pharmazeutisch verträgliche Salze.
10. Verbindungen der allgemeinen Formel IV

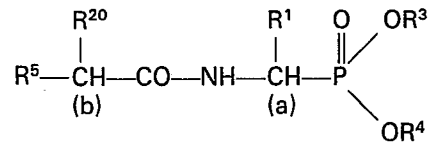

(IV)

worin $R^1$ wie in Formel I definiert ist; $R^3$ und $R^4$ Wasserstoff oder niedere Alkylschutzgruppen bedeuten, $R^5$ eine geschützte Aminogruppe darstellt und $R^{20}$ dasselbe wie $R^2$ in Formel I oder geschütztes 4-Guanidinobutyl darstellt, mit Konfigurationen an den C-Atomen (a) und (b) gemäß Anspruch 1.

11. (1R)-1-[(N-Benzyloxycarbonyl-L-norvalyl)-amino]-äthylphosphonsäure.
12. (1R)-1-[(N-Benzyloxycarbonyl-$\omega$-nitro-L-homoarginyl)-amino]-äthylphosphonsäure.
13. Aminosäurederivate gemäss den Ansprüchen 1—9 als pharmazeutische Wirkstoffe.
14. Aminosäurederivate gemäss den Ansprüchen 1—9 als antibakterielle Wirkstoffe bzw. als Mittel zur Potenzierung der Wirkung von Antibiotika.
15. Verfahren zur Herstellung von Verbindungen der allgemeinen Formel I gemäss Anspruch 1 und von deren pharmazeutisch verträglichen Salzen, gekennzeichnet durch Kondensation einer Verbindung der allgemeinen Formel II

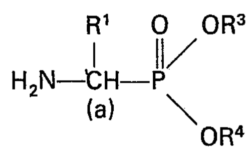

(II)

worin $R^1$, $R^3$, $R^4$ und (a) die in Anspruch 10 genannten Bedeutungen haben, mit einer geschützten $\alpha$-Aminosäure der allgemeinen Formel III

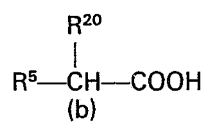

(III)

worin $R^5$, $R^{20}$ und (b) dasselbe wie in Formel IV bedeuten, gefolgt von Abspaltung vorhandener Schutzgruppen aus dem Kondensationsprodukt und, gewünschtenfalls, Ueberführung in ein pharmazeutisch verträgliches Salz.

16. Arzneimittel, enthaltend eine Verbindung der allgemeinen Formel I gemäss Ansprüchen 1—9 oder ein pharmazeutisch verträgliches Salz einer solchen Verbindung.
17. Antibakterielles Mittel, enthaltend eine Verbindung der allgemeinen Formel I gemäss Ansprüchen 1—9 oder ein Pharmazeutisch verträgliches Salz einer solchen Verbindung.
18. Antibakterielles Mittel, enthaltend eine Verbindung der allgemeinen Formel I gemäss Ansprüchen 1—9 oder ein pharmazeutisch verträgliches Salz einer solchen Verbindung sowie ein Antibiotikum.

## Revendications

1. Dérivés d'amino-acides de formule générale

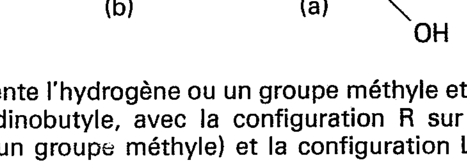

(I)

dans laquelle $R^1$ représente l'hydrogène ou un groupe méthyle et $R^2$ représente un groupe n-alkyle en $C_2$—$C_4$ ou 4-quanidinobutyle, avec la configuration R sur l'atome de carbone marqué (a) (lorsque $R^1$ représente un groupe méthyle) et la configuration L sur l'atome de carbone marqué (b),
et leurs sels acceptables pour l'usage pharmaceutique.

2. L'acide (1R)-1-[L-($\alpha$-aminobutyryl)-amino]éthylphosphonique et ses sels acceptables pour

l'usage pharmaceutique.

3. L'acide (1R)-1-(L-norleucylamino)-éthylphosphonique et ses sels acceptables pour l'usage pharmaceutique.

4. L'acide (1R)-1-(L-norvalylamino)-éthylphosphonique et ses sels acceptables pour l'usage pharmaceutique.

5. L'acide (L-$\alpha$-aminobutyrylamino)-méthylphosphonique et ses sels acceptables pour l'usage pharmaceutique.

6. L'acide (L-norleucylamino)-méthylphosphonique et ses sels acceptables pour l'usage pharmaceutique.

7. L'acide (L-norvalylamino)-méthylphosphonique et ses sels acceptables pour l'usage pharmaceutique.

8. L'acide (1R)-(L-homoarginylamino)-éthylphosphonique et ses sels acceptables pour l'usage pharmaceutique.

9. L'acide (L-homoarginylamino)-méthylphosphonique et ses sels acceptables pour l'usage pharmaceutique.

10. Composés de formule générale IV

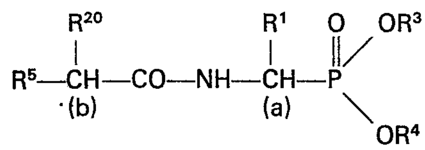

(IV)

dans laquelle $R^1$ a les significations indiquées en référence à la formule I; $R^3$ et $R^4$ représentent un atome d'hydrogène ou un groupe protecteur alkyle inférieur, $R^5$ représente un groupe amino protégé et $R^{20}$ a la même signification que $R^2$ dans la formule I ou représente un groupe 4-guanidinobutyle protégé, avec les configurations indiquées dans la revendication 1 sur les atomes de carbone (a) et (b).

11. L'acide (1R)-1-[(N-benzyloxycarbonyl-L-norvalyl)-amino]-éthylphosphonique.

12. L'acide (1R)-1-[(N-benzyloxycarbonyl-$\omega$-nitro-L-homoarginyl)-amino]-éthylphosphonique.

13. Dérivés d'amino-acides selon les revendications 1 à 9 en tant que substances actives pharmaceutiques.

14. Dérivés d'amino-acides selon les revendications 1 à 9 en tant que substances actives anti-bactériennes ou en tant que produits renforçant l'action des antibiotiques.

15. Procédé de préparation des composés de formule générale I selon la revendication 1 et de leurs sels acceptables pour l'usage pharmaceutique, caractérisé en ce que l'on condense un composé de formule générale II

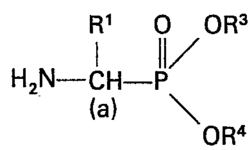

(II)

dans laquelle $R^1$, $R^3$, $R^4$ et (a) ont les significations indiquées dans la revendication 10, avec un $\alpha$-aminoacide protégé de formule générale III

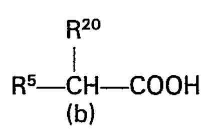

(III)

dans laquelle $R^5$, $R^{20}$ et (b) ont les significations indiquées en réference à la formule IV, après quoi on élimine du produit de condensation les groupes protecteurs éventuels et si on le désire, on convertit en un sel acceptable pour l'usage pharmaceutique.

16. Médicament contenant un composé de formule générale I selon l'une des revendications 1—9 ou un sel d'un tel composé acceptable pour l'usage pharmaceutique.

17. Produit anti-bactérien contenant un composé de formule générale I selon l'une des revendications 1—9 ou un sel d'un tel composé acceptable pour l'usage pharmaceutique.

18. Produit anti-bactérien contenant un composé de formule générale I selon l'une des revendications 1—9 ou un sel d'un tel composé acceptable pour l'usage pharmaceutique et un antibiotique.

**Claims**

1. Amino acid derivatives of general formula I

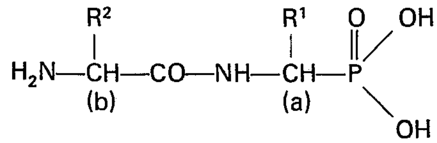

(I)

wherein $R^1$ signifies hydrogen or methyl and $R^2$ signifies n-$C_{2-4}$-alkyl or 4-guanidinobutyl, with the R-configuration at the C-atom denoted by (a) (when $R^1$ = methyl) and the L-configuration at the C-atom denoted by (b),
and their pharmaceutically compatible salts.

2. (1R)-1-[L-($\alpha$-Aminobutyryl)-amino]-ethylphosphonic acid and its pharmaceutically compatible salts.

3. (1R)-1-(L-Norleucylamino)-ethylphosphonic acid and its pharmaceutically compatible salts.

4. (1R)-1-(L-Norvalylamino)-ethylphosphonic acid and its pharmaceutically compatible salts.

5. (L-$\alpha$-Aminobutyrylamino)-methylphosphonic acid and its pharmaceutically compatible salts.

6. (L-Norleucylamino-)methylphosphonic acid and its pharmaceutically compatible salts.

7. (L-Norvalylamino)-methylphosphonic acid and its pharmaceutically compatible salts.

8. (1R)-1-(L-Homoarginylamino)-ethylphosphonic acid and its pharmaceutically compatible salts.

9. (L-Homoarginylamino)-methylphosphonic acid and its pharmaceutically compatible salts.

10. Compounds of general formula IV

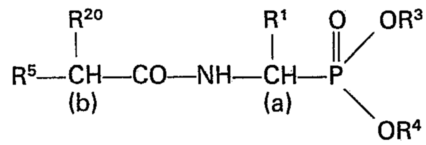

(IV)

wherein $R^1$ is defined as in formula I; $R^3$ and $R^4$ signify hydrogen or lower alkyl protecting groups; $R^5$ signifies a protected amino group and $R^{20}$ represents the same as $R^2$ in formula I or protected 4-guanidinobutyl, with configurations at the C-atoms (a) and (b) in accordance with claim 1.

11. (1R)-1-[(N-Benzyloxycarbonyl-L-norvalyl)-amino]-ethylphosphonic acid.

12. (1R)-1-[(N-Benzyloxycarbonyl-$\omega$-nitro-L-homoarginyl)-amino]-ethylphosphonic acid.

13. Amino acid derivatives in accordance with claims 1—9 as pharmaceutically active substances.

14. Amino acid derivatives in accordance with claims 1—9 as antibacterial active substances or as agents for potentiating the activity of antibiotics.

15. A process for the manufacture of compounds of general formula I in accordance with claim 1 and of their pharmaceutically compatible salts, characterized by the condensation of a compound of general formula II

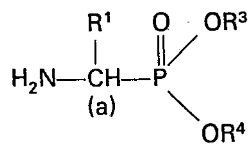

(II)

wherein $R^1$, $R^3$, $R^4$ and (a) have the significances set forth in claim 10,
with a protected $\alpha$-amino acid of general formula III

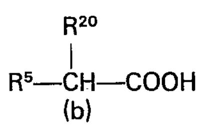

(III)

wherein $R^5$, $R^{20}$ and (b) signify the same as in formula IV,
followed by cleavage from the condensation product of protecting groups present and, if desired, conversion into a pharmaceutically compatible salt.

16. A medicament, containing a compound of general formula I in accordance with claims 1—9 or a pharmaceutically compatible salt of such a compound.

17. An antibacterial medicament, containing a compound of general formula I in accordance with claims 1—9 or a pharmaceutically compatible salt of such a compound.

18. An antibacterial medicament, containing a compound of general formula I in accordance with claims 1—9 or a pharmaceutically compatible salt of such a compound as well as an antibiotic.